# EUROPEAN PATENT APPLICATION

(11) **EP 1 890 149 A2**
(43) Date of publication of application: **20.02.2008**
(21) Application number: 07018323.1
(22) Date of filing: 29.10.2003
(51) Int. Cl.: G01N 33/558

(54) **Self-calibrated flow-through assay devices**

(30) Priority: 19.12.2002 US 325429
(62) Divisional of application: 03781542.0
(71) Applicant: KIMBERLY-CLARK WORLDWIDE, INC., Neenah, Wisconsin 54957-0349 (US)
(72) Inventor: Wei, Ning, Roswell, GA 30075 (US); Huang, Yanbin, Roswell, GA 30076 (US); Song, Xuedong, Roswell, GA 30076 (US); Kaylor, Rosann, Cumming, GA 30041 (US)
(74) Representative: Grünecker, Kinkeldey, Stockmair & Schwanhäusser Anwaltssozietät

(57) **Abstract**

A flow-through assay device capable of detecting the presence or quantity of an analyte residing in a test sample, said flow-through assay device comprising a porous membrane, said porous membrane being in communication with detection probes capable of generating a detection signal and calibration probes capable of generating a calibration signal, wherein the amount of the analyte is capable of determination from said detection signal as calibrated by said calibration signal, wherein said detection probes, said calibration probes, or combinations thereof, are conjugated with a specific binding member for the analyte, wherein said porous membrane defines a detection/calibration zone within which a polyelectrolyte capture reagent is immobilized that is configured to directly or indirectly bind to the calibration probes, and wherein a second capture reagent is immobilized on said porous membrane within said detection/calibration zone, wherein said second capture reagent is configured to directly or indirectly bind to said detection probes, wherein said detection probes are capable of generating a detection signal and said calibration probes are capable of generating a calibration signal while within said detection/calibration zone.

## Description

### Background of the Invention

Various analytical procedures and devices are commonly employed in flow-through assays to determine the presence and/or concentration of analytes that may be present in a test sample. For instance, immunoassays utilize mechanisms of the immune systems, wherein antibodies are produced in response to the presence of antigens that are pathogenic or foreign to the organisms. These antibodies and antigens, i.e., immunoreactants, are capable of binding with one another, thereby causing a highly specific reaction mechanism that can be used to determine the presence or concentration of that particular antigen in a biological sample.

There are several well-known immunoassay methods that use immunoreactants labeled with a detectable component so that the analyte can be detected analytically. For example, "sandwich-type" assays typically involve mixing the test sample with detectable probes, such as dyed latex or a radioisotope, which are conjugated with a specific binding member for the analyte. The conjugated probes form complexes with the analyte. These complexes then reach a zone of immobilized antibodies where binding occurs between the antibodies and the analyte to form ternary "sandwich complexes." The sandwich complexes are localized at the zone for detection of the analyte. This technique can be used to obtain quantitative or semi-quantitative results. Some examples of such sandwich-type assays are described in U.S. Patent Nos. 4,168,146 to Grubb, et al. and 4,366,241 to Tom, et al.

An alternative technique is the "competitive-type" assay. In a "competitive-type" assay, the label is typically a labeled analyte or analyte-analogue that competes for binding of an antibody with any unlabeled analyte present in the sample. Competitive assays are typically used for detection of analytes such as haptens, each hapten being monovalent and capable of binding only one antibody molecule. Examples of competitive immunoassay devices are described in U.S. Patent Nos. 4,235,601 to Deutsch, et al., 4,442,204 to Liotta, and 5,208,535 to Buechler, et al.

Many of these assays rely upon calibration to provide valid and meaningful results, particularly for semi-quantitative and quantitative detections. Specifically, either external or internal calibration systems may generally be employed. In an external calibration system, a standard curve can be obtained from standard samples containing a series of a known amount of analyte, and the results obtained from the samples are then compared with the standard curve to extract the presence and/or amount of the analyte in the sample. The external calibration method is relatively easy to design and simple to implement. However, it is often subject to interference from environmental and batch-to-batch variations, and is thus unreliable.

Conventional internal calibration systems, on the other hand, typically utilize a membrane that has a calibration zone and a detection zone on which the capturing reagent specific for the analyte is immobilized. Unfortunately, the ability of the calibration zone to provide a reliable and accurate comparison to the detection zone is often limited. Moreover, most internal calibration zones are relatively expensive, thereby making them impractical for certain applications.

As such, a need currently exists for an accurate calibration system for flow-through assays that is accurate and inexpensive.

### Summary of the Invention

In accordance with one embodiment of the present invention, a flow-through assay device (e.g., sandwich, competitive, etc.) is disclosed that is capable of detecting the presence or quantity of an analyte residing in a test sample. The assay device comprises a porous membrane that is in communication with detection probes and calibration probes that are capable of generating a detection signal and calibration signal, respectively. For example, in some embodiments, the probes are selected from the group consisting of chromogens, catalysts, fluorescent compounds, chemiluminescent compounds, phosphorescent compounds, radioactive compounds, direct visual labels, liposomes, and combinations thereof. In one particular embodiment, the probes contain a latex microparticle.

The porous membrane defines a detection/calibration zone within which a polyelectrolyte capture reagent is immobilized that is configured to directly or indirectly bind to the detection probes, the calibration probes, or combinations thereof. A second capture reagent that is a specific binding member for the analyte may be utilized. The detection/calibration zone is capable of generating a detection and calibration signal so that the amount of the analyte is capable of determination from the detection signal as calibrated by the calibration signal.

In accordance with another embodiment of the present invention, a method for detecting the presence or quantity of an analyte residing in a test sample is disclosed. The method comprises providing a flow-through assay device. A test sample containing the analyte is contacted with detection probes and calibration probes present with the device. At a detection/calibration zone, the intensity of the detection signal and calibration signal are measured. The amount of the analyte within the test sample is proportional to the intensity of the detection signal calibrated by the intensity of the calibration signal.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figures in which:
Fig. 1 is a perspective view of one embodiment of a flow-through assay device of the present invention;
Fig. 2 is a graphical illustration of one embodiment for covalently conjugating an antibody to carboxylated nanoparticles;
Fig. 3 is a schematic illustration of one embodiment of a flow-through assay device of the present invention;
Fig. 4 is a schematic illustration of another embodiment of a flow-through assay device of the present invention;
Fig. 5 is a schematic illustration of still another embodiment of a flow-through assay device of the present invention; and
Fig. 6 is a schematic illustration of another embodiment of a flow-through assay device of the present invention.

Repeat use of reference characters in the present specification and drawings is intended to represent same or analogous features or elements of the invention.

### Detailed Description of Representative Embodiments

### Definitions

As used herein, the term "analyte" generally refers to a substance to be detected. For instance, analytes can include antigenic substances, haptens, antibodies, and combinations thereof. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), drug intermediaries or byproducts, bacteria, virus particles and metabolites of or antibodies to any of the above substances. Specific examples of some analytes include ferritin; creatinine kinase MIB (CK-MB); digoxin; phenytoin; phenobarbitol; carbamazepine; vancomycin; gentamycin; theophylline; valproic acid; quinidine; leutinizing hormone (LH); follicle stimulating hormone (FSH); estradiol, progesterone; C-reactive protein; lipocalins; IgE antibodies; vitamin B2 micro-globulin; glycated hemoglobin (Gly. Hb); cortisol; digitoxin; N-acetylprocainamide (NAPA); procainamide; antibodies to rubella, such as rubella-IgG and rubella IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B e antigen (HBeAg); antibodies to hepatitis B e antigen (Anti-HBe); thyroid stimulating hormone (TSH); thyroxine (T4); total triiodothyronine (Total T3); free triiodothyronine (Free T3); carcinoembryoic antigen (CEA); and alpha fetal protein (AFP). Drugs of abuse and controlled substances include, but are not intended to be limited to, amphetamine; methamphetamine; barbiturates, such as amobarbital, secobarbital, pentobarbital, phenobarbital, and barbital; benzodiazepines, such as librium and valium; cannabinoids, such as hashish and marijuana; cocaine; fentanyl; LSD; methaqualone; opiates, such as heroin, morphine, codeine, hydromorphone, hydrocodone, methadone, oxycodone, oxymorphone and opium; phencyclidine; and propoxyhene. Other potential analytes may be described in U.S. Patent Nos. 6,436,651 to Everhart, et al. and 4,366,241 to Tom et al.

As used herein, the term "test sample" generally refers to a material suspected of containing the analyte. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, raucous, synovial fluid, peritoneal fluid, amniotic fluid or the like. The test sample can be pretreated prior to use, such as preparing plasma from blood, diluting viscous fluids, and the like. Methods of treatment can involve filtration, distillation, concentration, inactivation of interfering components, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.

### Detailed Description

Reference now will be made in detail to various embodiments of the invention, one or more examples of which are set forth below. Each example is provided by way of explanation of the invention, not limitation of the invention. In fact, it will be apparent to those skilled in the art that various modifications and variations can be made in the present invention without departing from the scope or spirit of the invention. For instance, features illustrated or described as part of one embodiment, can be used on another embodiment to yield a still further embodiment. Thus, it is intended that the present invention covers such modifications and variations as come within the scope of the appended claims and their equivalents.

In general, the present invention is directed to an internal, self-calibrated system for flow-through assay devices. In particular, the present invention employs the use of a single detection/calibration zone defined by a porous membrane of the assay. Conducting signal detection and internal calibration at the same time can greatly increase the detection reliability and reproducibility by eliminating the influences of environmental factors, such as temperature, pH and instrumental instability of the detection signal.

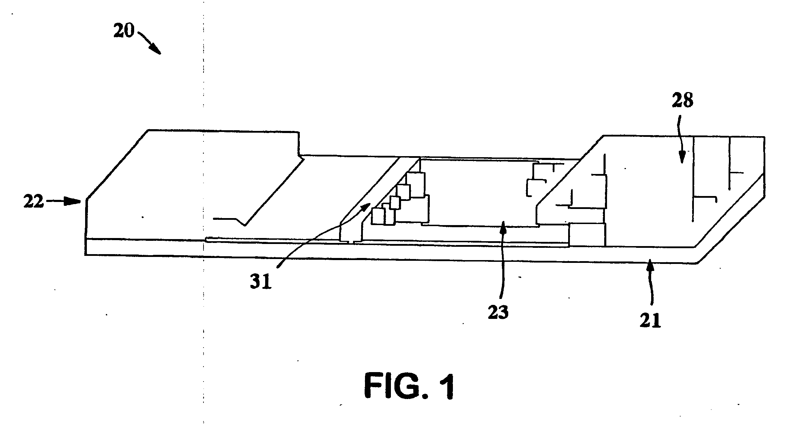

Referring to Fig. 1, for instance, one embodiment of a flow-through assay device 20 that can be formed according to the present invention will now be described in more detail. As shown, the device 20 contains a porous membrane 23 optionally supported by a rigid material 21. In general, the porous membrane 23 can be made from any of a variety of materials through which the test sample is capable of passing. For example, the materials used to form the porous membrane 23 can include, but are not limited to, natural, synthetic, or naturally occurring materials that are synthetically modified, such as polysaccharides (e.g., cellulose materials such as paper and cellulose derivatives, such as cellulose acetate and nitrocellulose); silica; inorganic materials, such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon or rayon); porous gels, such as silica gel, agarose, dextran, and gelatin; polymeric films, such as polyacrylamide; and the like. In one particular embodiment, the porous membrane 23 is formed from nitrocellulose and/or polyester sulfone materials. It should be understood that the term "nitrocellulose" refers to nitric acid esters of cellulose, which may be nitrocellulose alone, or a mixed ester of nitric acid and other acids, such as aliphatic carboxylic acids having from 1 to 7 carbon atoms.

The device 20 may also contain a wicking pad 28. The wicking pad 28 generally receives fluid that has migrated through the entire porous membrane 23. As is well known in the art, the wicking pad 28 can assist in promoting capillary action and fluid flow through the membrane 23.

To initiate the detection of an analyte within the test sample, a user may directly apply the test sample to a portion of the porous membrane 23 through which it can then travel to reach a detection/calibration zone 31 (described below). Alternatively, the test sample may first be applied to a sampling pad (not shown) that is in fluid communication with the porous membrane 23. Some suitable materials that can be used to form the sampling pad include, but are not limited to, nitrocellulose, cellulose, porous polyethylene pads, and glass fiber filter paper. If desired, the sampling pad may also contain one or more assay pretreatment reagents, either diffusively or non-diffusively attached thereto.

In the illustrated embodiment, the test sample travels from the sampling pad (not shown) to a conjugate pad 22 that is placed in communication with one end of the sampling pad. The conjugate pad 22 is formed from a material through which the test sample is capable of passing. For example, in one embodiment, the conjugate pad 22 is formed from glass fibers. Although only one conjugate pad 22 is shown, it should be understood that other conjugate pads may also be used in the present invention.

To facilitate accurate detection of the presence or absence of an analyte within the test sample, probes are applied at various locations of the device 20. As will be described in more detail below, probes are used for both detection of the analyte and for calibration. Any substance generally capable of generating a signal that is detectable visually or by an instrumental device may be used as probes. Various suitable substances can include chromogens; catalysts; fluorescent compounds; chemiluminescent compounds; phosphorescent compounds; radioactive compounds; direct visual labels, including colloidal metallic (e.g., gold) and non-metallic particles, dye particles, enzymes or substrates, or organic polymer latex particles; liposomes or other vesicles containing signal producing substances; and the like. For instance, some enzymes suitable for use as probes are disclosed in U.S. Patent No. 4,275,149 to Litman, et al., which is incorporated herein in its entirety by reference thereto for all purposes. One example of an enzyme/substrate system is the enzyme alkaline phosphatase and the substrate nitro blue tetrazolium-5-bromo-4-chloro-3-indolyl phosphate, or derivative or analog thereof, or the substrate 4-methylumbelliferyl-phosphate. Other suitable probes may be described in U.S. Patent Nos. 5,670,381 to Jou, et al. and 5,252,459 to Tarcha, et al., which are incorporated herein in their entirety by reference thereto for all purposes.

In some embodiments, the probes can contain a fluorescent compound that produces a detectable signal. The fluorescent compounds can be fluorescent molecules, polymers, dendrimers, particles, and the like. Some examples of suitable fluorescent molecules, for instance, include, but are not limited to, fluorescein, europium chelates, phycobiliprotein, rhodamine and their derivatives and analogs. A visually detectable, colored compound can also be used as a probe, thereby providing for a direct colored readout of the presence or concentration of the analyte in the sample without the need for further signal producing reagents.

The probes, such as described above, may be used alone or in conjunction with a microparticle (sometimes referred to as "beads" or "microbeads"). For instance, naturally occurring microparticles, such as nuclei, mycoplasma, plasmids, plastids, mammalian cells (e.g., erythrocyte ghosts), unicellular microorganisms (e.g., bacteria), polysaccharides (e.g., agarose), and the like, can be used. Further, synthetic microparticles may also be utilized. For example, in one embodiment, latex microparticles that are labeled with a fluorescent or colored dye are utilized. Although any latex microparticle may be used in the present invention, the latex microparticles are typically formed from polystyrene, butadiene styrenes, styreneacrylic-vinyl terpolymer, polymethylmethacrylate, polyethylmethacrylate, styrene-maleic anhydride copolymer, polyvinyl acetate, polyvinylpyridine, polydivinylbenzene, polybutyleneterephthalate, acrylonitrile, vinylchloride-acrylates, and the like, or an aldehyde, carboxyl, amino, hydroxyl, or hydrazide derivative thereof. Other suitable microparticles may be described in U.S. Patent Nos. 5,670,381 to Jou, et al. and 5,252,459 to Tarcha, et al., which are incorporated herein in their entirety by reference thereto for all purposes. Some commercially available examples of suitable fluorescent particles include fluorescent carboxylated microspheres sold by Molecular Probes, Inc. under the trade names "FluoSphere" (Red 580/605) and "TransfluoSphere" (543/620), as well as "Texas Red" and 5- and 6-carboxytetramethylrhodamine, which are also sold by Molecular Probes, Inc. Commercially available examples of suitable colored, latex microparticles include carboxylated latex beads sold by Bang's Laboratory, Inc.

When the probes are particles, such as described above, the mean diameter of the particles may generally vary as desired depending on factors such as the type of particle chosen, the pore size of the membrane, and the membrane composition. For example, in some embodiments, the mean diameter of the particulate labels can range from about 0.01 microns to about 1,000 microns, in some embodiments from about 0.01 microns to about 100 microns, and in some embodiments, from about 0.01 microns to about 10 microns. In one particular embodiment, the particles have a mean diameter of from about 0.1 to about 2 microns. Generally, the particles are substantially spherical in shape, although other shapes including, but not limited to, plates, rods, bars, irregular shapes, etc., are suitable for use in the present invention. As will be appreciated by those skilled in the art, the composition, shape, size, and/or density of the particles may widely vary.

In some instances, it is desired to modify the probes in some manner so that they are more readily able to bond to the analyte. In such instances, the probes can be modified with certain specific binding members that are adhered thereto to form probe conjugates. Specific binding members generally refer to a member of a specific binding pair, i.e., two different molecules where one of the molecules chemically and/or physically binds to the second molecule. For instance, immunoreactive specific binding members can include antigens, haptens, aptamers, antibodies, and complexes thereof, including those formed by recombinant DNA methods or peptide synthesis. An antibody can be a monoclonal or polyclonal antibody, a recombinant protein or a mixture(s) or fragment(s) thereof, as well as a mixture of an antibody and other specific binding members. The details of the preparation of such antibodies and their suitability for use as specific binding members are well known to those skilled in the art. Other common specific binding pairs include but are not limited to, biotin and avidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Furthermore, specific binding pairs can include members that are analogs of the original specific binding member. For example, a derivative or fragment of the analyte, i.e., an analyte-analog, can be used so long as it has at least one epitope in common with the analyte.

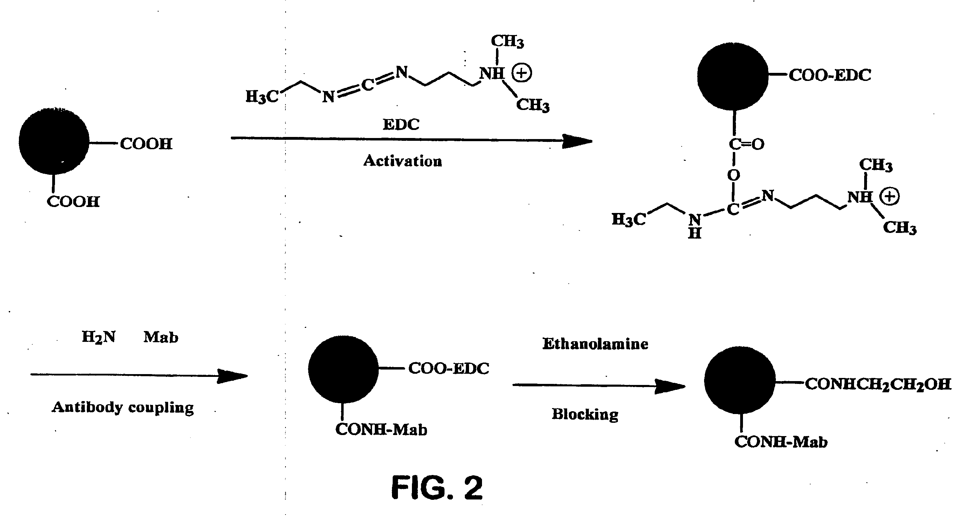

The specific binding members can generally be attached to the probes using any of a variety of well-known techniques. For instance, covalent attachment of the specific binding members to the probes (e.g., microparticles) can be accomplished using carboxylic, amino, aldehyde, bromoacetyl, iodoacetyl, thiol, epoxy and other reactive or linking functional groups, as well as residual free radicals and radical cations, through which a protein coupling reaction can be accomplished. A surface functional group can also be incorporated as a functionalized co-monomer because the surface of the microparticle can contain a relatively high surface concentration of polar groups. In addition, although microparticle probes are often functionalized after synthesis, in certain cases, such as poly(thiophenol), the microparticles are capable of direct covalent linking with a protein without the need for further modification. For example, referring to Fig. 2, one embodiment of the present invention for covalently conjugating a probe is illustrated. As shown, the first step of conjugation is activation of carboxylic groups on the probe surface using carbodiimide. In the second step, the activated carboxylic acid groups are reacted with an amino group of an antibody to form an amide bond. The activation and/or antibody coupling can occur in a buffer, such as phosphate-buffered saline (PBS) (e.g., pH of 7.2) or 2-(N-morpholino) ethane sulfonic acid (MES) (e.g., pH of 5.3). As shown, the resulting probes can then be blocked with ethanolamine, for instance, to form the probe conjugate. Besides covalent bonding, other attachment techniques, such as physical adsorption, may also be utilized in the present invention.

Referring again to Fig. 1, the device 20 also contains the detection/calibration zone 31. The detection/calibration zone 31 generally provides a single distinct detection/calibration region (e.g., line, dot, etc.) so that a user can accurately determine the concentration of a particular analyte within a test sample at one location along the device. For instance, in the illustrated embodiment, a single line is utilized.

Further, the line may be disposed in a direction that is substantially perpendicular to the flow of the test sample through the device 20. Likewise, in some embodiments, the line may be disposed in a direction that is substantially parallel to the flow of the test sample through the device 20.

The detection/calibration zone 31 contains one or more immobilized capture reagents that are capable of binding to the probes, either directly or indirectly (i.e., to a specific binding member conjugated to the probe, to an analyte complexed with the probe, etc.). In some embodiments, a first immobilized capture reagent is configured to bind to the detection probes, while a second capture reagent is configured to bind to the calibration probes. The first and second immobilized capture reagents may be the same or different.

Generally speaking, various types of capture reagents may be immobilized within the detection/calibration zone 31. For example, in one embodiment, the capture reagent may be identical to or formed from the same class or category of materials (e.g., antibodies, antigens, analogs thereof, and the like) as the specific binding members used to form conjugates of the probes. Thus, in this embodiment, the capture reagent can act as a stationary binding site for the probes when bound to an analyte. Specifically, the analyte, such as an antibody, antigen, etc., can have two binding sites. Upon reaching the detection/calibration zone 31 and passing through the conjugate pad 22, one of these binding sites is occupied by the specific binding member conjugated to the probe. However, the free binding site of the analyte can bind to the immobilized capture reagent.

In another embodiment, the immobilized capture reagent may be a polyelectrolyte that binds to the probes, either directly or indirectly, through ionic interaction. For instance, the polyelectrolyte can have a net positive or negative charge, as well as a net charge that is generally neutral. Some suitable examples of polyelectrolytes having a net positive charge include, but are not limited to, polylysine (commercially available from Sigma-Aldrich Chemical Co., Inc. of St.. Louis, Missouri), polyethylenimine; epichlorohydrin-functionalized polyamines and/or polyamidoamines, such as poly(dimethylamine-co-epichlorohydrin); polydiallyldimethyl-ammonium chloride; cationic cellulose derivatives, such as cellulose copolymers or cellulose derivatives grafted with a quaternary ammonium water-soluble monomer; and the like. In one particular embodiment, CelQuat® SC-230M or H-100 (available from National Starch & Chemical, Inc.), which are cellulosic derivatives containing a quaternary ammonium water-soluble monomer, can be utilized. Moreover, some suitable examples of polyelectrolytes having a net negative charge include, but are not limited to, polyacrylic acids, such as poly(ethylene-co-methacrylic acid, sodium salt), and the like. It should also be understood that other polyelectrolytes may also be utilized in the present invention, such as amphiphilic polyelectrolytes (i.e., having polar and non-polar portions). For instance, some examples of suitable amphiphilic polyelectrolytes include, but are not limited to, poly(styryl-b-N-methyl 2-vinyl pyridinium iodide) and poly(styryl-b-acrylic acid), both of which are available from Polymer Source, Inc. of Dorval, Canada.

Although any polyelectrolyte may generally be used, the polyelectrolyte selected for a particular application may vary depending on the nature of the probes, specific binding members, the analyte of interest, the type of porous membrane, and the like. In particular, the distributed charge of a polyelectrolyte allows it to bind to substances having an opposite charge. Thus, for example, polyelectrolytes having a net positive charge are often better equipped to bind with probes that are negatively charged, while polyelectrolytes that have a net negative charge are often better equipped to bind to probes that are positively charged. Thus, in such instances, the ionic interaction between these molecules allows the required binding to occur within the detection/calibration zone 31. Nevertheless, although ionic interaction is primarily utilized to achieve the desired binding, polyelectrolytes can also bind with probes having a similar charge.

Because the polyelectrolyte is designed to bind to the probes, it is typically desired that the polyelectrolyte be substantially non-diffusively immobilized on the surface of the porous membrane 23. Thus, the polyelectrolytes can be applied to the porous membrane 23 in such a manner that the polyelectrolytes do not substantially diffuse into the matrix of the porous membrane 23. In particular, the polyelectrolytes typically form an ionic and/or covalent bond with functional groups present on the surface of the porous membrane 23 so that they remain immobilized thereon. Although not required, the formation of covalent bonds between the polyelectrolyte and the porous membrane 23 may be desired to more permanently immobilize the polyelectrolyte thereon.

For example, in one embodiment, the monomers used to form the polyelectrolyte are first formed into a solution and then applied directly to the porous membrane 23. Various solvents (e.g., organic solvents, water, etc.) may be utilized to form the solution. Once applied, the polymerization of the monomers is initiated using heat, electron beam radiation, free radical polymerization, and the like. In some instances, as the monomers polymerize, they form covalent bonds with certain functional groups of the porous membrane 23, thereby immobilizing the resulting polyelectrolyte thereon. For example, in one embodiment, an ethyleneimine monomer can form a covalent bond with a carboxyl group present on the surface of some porous membranes (e.g., nitrocellulose).

In another embodiment, the polyelectrolyte can be formed prior to application to the porous membrane 23. If desired, the polyelectrolyte may first be formed into a solution using organic solvents, water, and the like. Thereafter, the polyelectrolytic solution is applied directly to the porous membrane 23 and then dried. Upon drying, the polyelectrolyte may, as described above, form an ionic bond with certain functional groups present on the surface of the porous membrane 23 that have a charge opposite to the polyelectrolyte. For example, in one embodiment, positively-charged polyethyleneimine can form an ionic bond with negatively-charged carboxyl groups present on the surface of some porous membranes (e.g., nitrocellulose).

In addition, the polyelectrolyte may also be crosslinked to the porous membrane 23 using various well-known techniques. For example, in some embodiments, epichlorohydrin-functionalized polyamines and/or polyamidoamines can be used as a crosslinkable, positively-charged polyelectrolyte. Examples of these materials are described in U.S. Pat. Nos. 3,700,623 to Keim; 3,772,076 to Keim; and 4,537,657 to Keim, which are incorporated herein in their entirety by reference thereto for all purposes and are believed to be sold by Hercules, Inc., Wilmington, Del. under the Kymene™ trade designation. For instance, Kymene™ 450 and 2064 are epichlorohydrin-functionalized polyamine and/or polyamidoamine compounds that contain epoxide rings and quaternary ammonium groups that can form covalent bonds with carboxyl groups present on certain types of porous membranes (e.g., nitrocellulose) and crosslink with the polymer backbone of the porous membrane when cured. In some embodiments, the crosslinking temperature can range from about 50°C to about 120°C and the crosslinking time can range from about 10 to about 600 seconds.

Although various techniques for non-diffusively immobilizing polyelectrolytes on the porous membrane 23 have been described above, it should be understood that any other technique for non-diffusively immobilizing polyelectrolytic compounds can be used in the present invention. In fact, the aforementioned methods are only intended to be exemplary of the techniques that can be used in the present invention. For example, in some embodiments, certain components may be added to the polyelectrolyte solution that can substantially inhibit the diffusion of such polyelectrolytes into the matrix of the porous membrane 23.

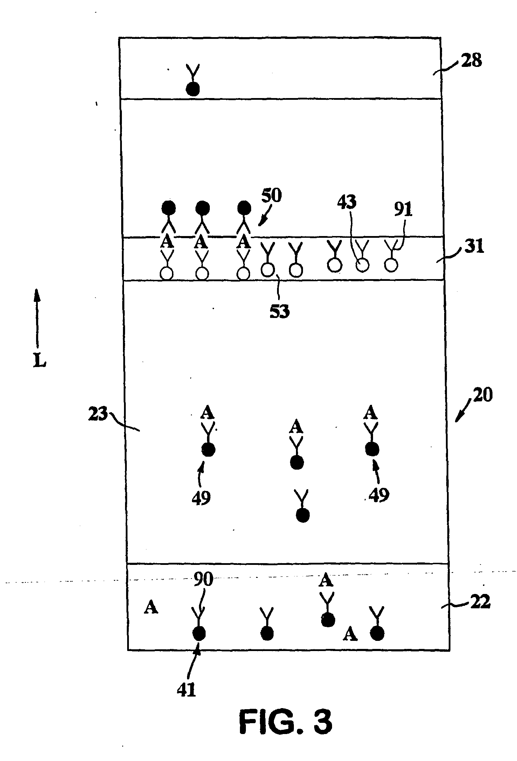

In general, a variety of flow-through assay devices may be constructed according to the present invention. In this regard, various embodiments of the present invention will now be described in more detail. It should be understood, however, that the embodiments discussed below are only exemplary, and that other embodiments are also contemplated by the present invention. For instance, referring to Fig. 3, one particular embodiment in which probes 41 are used for detection and probes 43 are used for calibration is shown. In this embodiment, the detection probes 41 are applied to the conjugate pad 22 and are thus capable of flowing through the device 20 (as indicated by the directional arrow L) when placed in communication with the test sample. The detection probes 41 are conjugated with a specific binding member 90 for an analyte A so that, upon contact with the analyte A, the probes 41 bind thereto to form analyte/probe complexes 49. The analyte/probe complexes 49 may then flow through the device 20 until they reach the detection/calibration zone 31.

Within the detection/calibration zone 31, a polyelectrolyte capture reagent is immobilized (not shown) for binding to calibration probes 43 through ionic interaction. In this embodiment, the calibration probes 43 are immobilized on the polyelectrolyte before the test sample is applied. The calibration probes 43 may include, for instance, latex microparticles conjugated with a specific binding member 91 (e.g., antibody, antigen, etc.). At the detection/calibration zone 31, the analyte/probe complexes 49 can bind to the specific binding member 91 of the calibration probes 43 to form a sandwich complex 50. Any unbound probes 41 will flow through the detection/calibration zone 31. If desired, the amount of the polyelectrolyte capture reagent may be predetermined so that the calibration signal within the detection/calibration zone 31 reaches its full and predetermined potential for signal intensity. That is, the amount of probes 43 that are deposited is predetermined because the amount of the polyelectrolyte employed is set at a predetermined and known level.

Once allowed to fully develop, the intensity level of the probes 43 may be used to calibrate the intensity level of the probes 41 at the detection/calibration zone 31 to determine the amount of analyte present in the test sample. This calibration step may occur visually, with the aid of a reading device, or using other techniques. For example, in one embodiment, the probes 41 and 43 may be labeled with fluorescent-producing compounds so that the signal intensity of the probes 41 and 43 may be measured using conventional techniques. For example, in one embodiment, the probes 41 and 43 can be excited with the same external source. In this embodiment, the source supplies radiation at an excitation wavelength, thereby causing the probes 41 to emit light at a wavelength that is different than the wavelength emitted by the probes 43. This enables the presence of the probes 41 and 43 to be separately measured. Alternatively, the probes 41 and 43 can also be measured separately using separate external sources.

Generally speaking, fluorescence is the result of a three-stage process that occurs in certain fluorescent compounds. In the first stage, energy is supplied by an external source, such as an incandescent lamp or a laser and absorbed by the fluorescent compound, creating an excited electronic singlet state. In the second stage, the excited state exists for a finite time during which the fluorescent compound undergoes conformational changes and is also subject to a multitude of possible interactions with its molecular environment. During this time, the energy of the excited state is partially dissipated, yielding a relaxed state from which fluorescence emission originates. The third stage is the fluorescence emission stage wherein energy is emitted, returning the fluorescent compound to its ground state. The emitted energy is lower than its excitation energy (light or laser) and thus of a longer wavelength. This shift or difference in energy or wavelength allows the emission energy to be detected and isolated from the excitation energy.

Fluorescence detection generally utilizes wavelength filtering to isolate the emission photons from the excitation photons, and a detector that registers emission photons and produces a recordable output, usually as an electrical signal or a photographic image. There are generally four recognized types of detectors:
spectrofluorometers and microplate readers; fluorescence microscopes; fluorescence scanners; and flow cytometers. One suitable fluorescence detector for use with the present invention is a FluoroLog III Spectrofluorometer, which is sold by SPEX Industries, Inc. of Edison, New Jersey.

Although not required, the selection criteria of particularly desired detection and calibration probe pairs include: (1) little or no spectral overlap for either the absorption spectra or the fluorescence spectra so that emission intensities can be measured separately; (2) no significant fluorescent energy transfer between the detection and calibration probes when brought into a close proximity so that they emit independently; and (3) relatively long emission wavelength (e.g., greater than about 600 nm) so that the autofluorescence of biological fluids has a minimal effect on the fluorescence measurement.

Further, if desired, a technique known as "time-resolved fluorescence detection" may also be utilized in the present invention. Time-resolved fluorescence detection is designed to reduce background signals from the emission source or from scattering processes (resulting from scattering of the excitation radiation) by taking advantage of the fluorescence characteristics of certain fluorescent materials, such as lanthanide chelates of europium (Eu (III)) and terbium (Tb (III)). Such chelates can exhibit strongly red-shifted, narrow-band, long-lived emission after excitation of the chelate at substantially shorter wavelengths. Typically, the chelate possesses a strong ultraviolet absorption band due to a chromophore located close to the lanthanide in the molecule. Subsequent to light absorption by the chromophore, the excitation energy can be transferred from the excited chromophore to the lanthanide. This is followed by a fluorescence emission characteristic of the lanthanide. The use of pulsed excitation and time-gated detection, combined with narrow-band emission filters, allows for specific detection of the fluorescence from the lanthanide chelate only, rejecting emission from other species present in the sample that are typically shorter-lived or have shorter wavelength emission. Other time-resolved techniques for measuring fluorescence are described in U.S. Patent No. 5,585,279 to Davidson and 5,637,509 to Hemmila, et al., which are incorporated herein in their entirety by reference thereto for all purposes.

Regardless of the technique used to measure the signal intensity of the probes 41 and 43, it has been discovered that the use of a single detection/calibration zone 31 can enable the detection and calibration probes 41 and 43 to be measured at the same location of the device 20. This "single measurement location" approach provides a simple and attractive approach for users of the assay device, particularly those targeted for home and point-of-care applications.

At the detection/calibration zone 31, the amount of the analyte can be ascertained from the signal intensity of the detection probes 41 as calibrated by the signal intensity of the calibration probes 43. Specifically, the total amount of the probes 43 is predetermined and known and thus can be used for calibration purposes. Thus, the amount of analyte in the test sample is directly proportional to Iₛ (signal intensity of the probes 41), while the signal intensity of the probes 43, I_{c}, should remain relatively constant regardless of the present of the analyte. Based upon the intensity range in which this calibrated value falls, the general concentration range for the analyte may be determined. As a result, calibration and sample testing may be conducted under approximately the same conditions at the same time, thus providing reliable quantitative or semi-quantitative results, with increased sensitivity.

If desired, the ratio of Iₛ to I_{c} may be plotted versus the analyte concentration for a range of known analyte concentrations to generate a calibration curve. To determine the quantity of analyte in an unknown test sample, the signal ratio may then be converted to analyte concentration according to the calibration curve. It should be noted that alternative mathematical relationships between Iₛ and I_{c} may be plotted versus the analyte concentration to generate the calibration curve. For example, in one embodiment, the value of Iₛ /(Iₛ + I_{c}) may be plotted versus analyte concentration to generate the calibration curve.

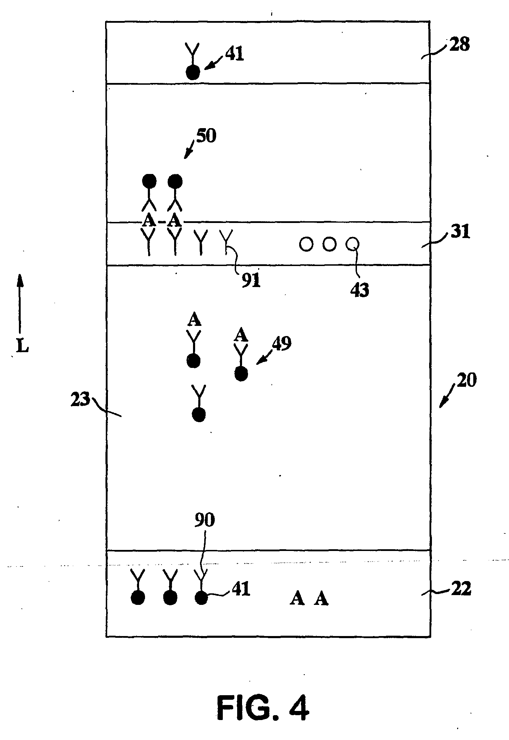

Referring to Fig. 4, another embodiment in which probes 41 are used for detection and probes 43 are used for calibration is shown. The detection probes 41 are applied to the conjugate pad 22 and are thus capable of flowing through the device 20 (as indicated by the directional arrow L) when placed in communication with the test sample. The detection probes 41 are conjugated with a specific binding member 90 for an analyte A so that, upon contact with the analyte A, the probes 41 bind thereto to form analyte/probe complexes 49. The analyte/probe complexes 49 may then flow through the device 20 until they reach the detection/calibration zone 31. The calibration probes 43 are immobilized within the detection/calibration zone 31 via a polyelectrolyte capture reagent. For example, the calibration probes 43 may include labeled microparticles, polymers, or molecules that bind to the polyelectrolyte through ionic interaction. In addition, a specific binding member 91 (e.g., antibody, antigen, etc.) is also separately immobilized within the detection/calibration zone 31. Based on the nature of the detection probes 41, neither the complexes 49, nor unbound probes 41, bind to the polyelectrolyte capture reagent. Instead, the analyte/probe complexes 49 bind to the specific binding member 91 to form complexes 50 and any unbound probes 41 flow through the detection/calibration zone 31. Once allowed to fully develop, the intensity signal Iₛ of the probes 41 at the detection/calibration zone 31 may be determined to calculate the amount of analyte present in the test sample. In this embodiment, the amount of analyte in the test sample is directly proportional to Iₛ. Moreover, the intensity signal I_{c} of the calibration probes 43 may also be used to calibrate Iₛ. For example, the ratio of Iₛ to I_{c} may be plotted versus the analyte concentration for a range of known analyte concentrations to generate a calibration curve.

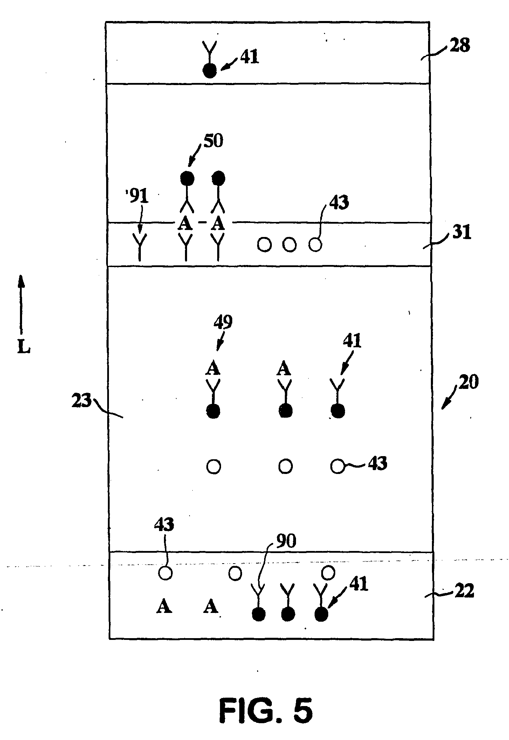

Referring to Fig. 5, still another embodiment in which probes 41 are used for detection and probes 43 are used for calibration is shown. The detection probes 41 and calibration probes 43 are applied to the conjugate pad 22 and are thus capable of flowing through the device 20 (as indicated by the directional arrow L) when placed in communication with the test sample. The detection probes 41 are conjugated with a specific binding member 90 for an analyte A so that, upon contact with the analyte A, the probes 41 bind thereto to form analyte/probe complexes 49. The analyte/probe complexes 49 may then flow through the device 20 until they reach the detection/calibration zone 31. A polyelectrolyte is immobilized within the detection/calibration zone 31 for selective binding with the calibration probes 43 (e.g., labeled microparticles, polymers, or molecules) through ionic interaction. Specifically, the probes 43 may be smaller in size than the probes 41 so that they arrive at the detection/calibration zone 31 before the probes 41 and occupy all of the binding sites provided by the polyelectrolyte. Alternatively, the charges of the probes 41 and 43 may be such that only the probes 43 bind to the polyelectrolyte. In any event, a specific binding member 91 (e.g., antibody, antigen, etc.) is also separately immobilized within the detection/calibration zone 31. At the detection/calibration zone 31, the analyte/probe complexes 49 can bind to the specific binding member 91 to form complexes 50. Any unbound probes 41 will flow through the detection/calibration zone 31. Once allowed to fully develop, the intensity signal Iₛ of the probes 41 at the detection/calibration zone 31 may be determined to calculate the amount of analyte present in the test sample. In this embodiment, the amount of analyte in the test sample is directly proportional to Iₛ. Moreover, the intensity signal I_{c} of the calibration probes 43 may also be used to calibrate Iₛ. For example, the ratio of Iₛ to I_{c} may be plotted versus the analyte concentration for a range of known analyte concentrations to generate a calibration curve.

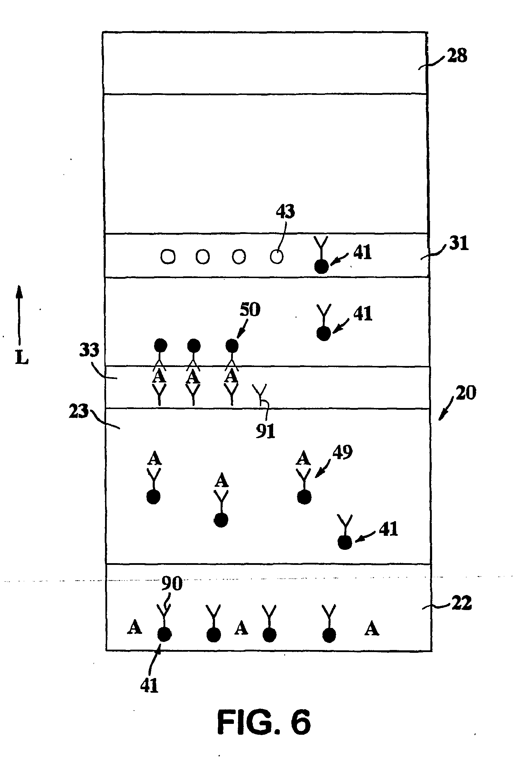

In some embodiments, the device 20 may contain additional zones that facilitate in the detection of the analyte of interest. For example, as shown in Fig. 6, the device 20 may contain a capture zone 33 that is positioned upstream from the detection/calibration zone 31. The capture zone 33 contains a first capture reagent that is specific to either the detection or the calibration probes. For instance, in the illustrated embodiment, detection probes 41 are applied to the conjugate pad 22 and are thus capable of flowing through the device 20 when placed in communication with the test sample. The detection probes 41 may be conjugated with a specific binding member 90 for an analyte A so that, upon contact with the analyte A, the probes 41 bind thereto to form analyte/probe complexes 49. The analyte/probe complexes 49 may then flow through the device 20 until they reach the capture zone 33. Specific binding members 91 such as an antibody or antigen that corresponds to the analyte A, are immobilized within the capture zone 33. Thus, at the capture zone 33, the analyte/probe complexes 49 can bind to the specific binding members 91 immobilized thereon to form complexes 50.

Any unbound probes 41 will then flow to the detection/calibration zone 31. In this embodiment, a second capture reagent (e.g., polyelectrolyte) is also immobilized within the detection/calibration zone 31 that is capable of binding to any unbound probes 41 through ionic interaction. Further, the calibration probes 43 may also be immobilized on the second capture reagent within the detection/calibration zone 31. For example, the calibration probes 43 may include latex microparticles that also bind to the polyelectrolyte through ionic interaction.

Once allowed to fully develop, the intensity signal Iₛ of the probes 41 at the detection/calibration zone 31 may be determined to calculate the amount of analyte present in the test sample. In this embodiment, the amount of analyte in the test sample is inversely proportional to Iₛ. Moreover, the intensity signal I_{c} of the calibration probes 43 may also be used to calibrate Iₛ. For example, the ratio of Iₛ to I_{c} may be plotted versus the analyte concentration for a range of known analyte concentrations to generate a calibration curve. To determine the quantity of analyte in an unknown test sample, the signal ratio may then be converted to analyte concentration according to the calibration curve. It should be noted that alternative mathematical relationships between I_{c} and Iₛ may be plotted versus the analyte concentration to generate the calibration curve.

Although various embodiments of assay configurations have been described above, it should be understood, that an assay of the present invention may generally have any configuration desired, and need not contain all of the components described above. Further, other well-known components of assays not specifically referred to herein may also be utilized in the present invention. For example, various assay configurations are described in U.S. Patent Nos. 5,395,754 to Lambotte, et al.; 5,670,381 to Jou, et al.; and 6,194,220 to Malick, et al., which are incorporated herein in their entirety by reference thereto for all purposes. In addition, it should also be understood that competitive assays may also be formed according to the present invention. Techniques and configurations of competitive assays are well known to those skilled in the art.

As an example, the flow-through device 20 described above and illustrated in Fig. 3 can be easily modified to form a competitive assay. In this embodiment, the detection probes 41 may be an analyte or analyte analog, while the calibration probes 43 may include, for instance, latex microparticles conjugated with a specific binding member for the analyte of interest. When the test sample is applied, the detection probes 41 travel through the porous membrane 23 until they reach the detection/calibration zone 31. At the zone 31, the detection probes 41 and any analyte A within the test sample compete for the specific binding members 91 (e.g., antibody, antigen, etc.). Any unbound probes 41 will flow through the detection/calibration zone 31. In this embodiment, the amount of analyte in the test sample is inversely proportional to Iₛ (signal intensity of the probes 41). If desired, the ratio of Iₛ to I_{c} (signal intensity of the probes 43) may be plotted versus the analyte concentration for a range of known analyte concentrations to generate a calibration curve.

The present invention may be better understood with reference to the following examples.

### EXAMPLE 1

The ability of an internal detection/calibration zone of the present invention to calibrate a sandwich assay was demonstrated. Initially, HF 120 porous membrane samples were laminated onto corresponding supporting cards having a length of approximately 30 centimeters. Fluorescent beads (calibration probes) were obtained from Molecular Probes, Inc. that had a particle size of 0.2 micrometers and excitation/emission wavelengths of 505/515 nanometers. The beads were washed and stored in a storage buffer at 0.28% weight percentage. 40 microliters of the fluorescent bead solution was mixed with 10 microliters of a polylysine solution and striped onto the membrane to form a detection/calibration line. Monoclonal antibody CRP Mab 5804 having a concentration of 2.36 milligrams per milliliter (obtained from Biogenesis, Inc.) was immobilized on the porous membrane samples to form a capture line. The membrane samples were then dried for 1 hour at a temperature of 37°C. A cellulosic fiber wicking pad (Millipore Co.) was attached to one end of the membrane and cut into 4-millimeter strips.

The half stick samples were put into various micro-wells in which mixtures of fluorescent beads (detection probes) and different concentrations of CRP antigen were contained. The amount of the fluorescent beads was the same for each mixture, i.e., 4 micrograms. The fluorescent detection beads were formed as follows. Initially, polystyrene beads having carboxyl functional groups (obtained from Molecular Probes, Inc.) were provided. The beads had a particle size of 0.5 micrometers and excitation/emission wavelengths of 580/605 nanometers. 100 microliters of the polystyrene beads (2% concentration) were washed twice with MES buffer and separated with a centrifuge. The pellitized beads were re-suspended in 100 microliters of MES buffer and mixed with 50 microliters of MES buffer containing 4 milligrams of carbodiimmide (from Polysciences, Inc.). The mixture was reacted at room temperature for 20 minutes on a shaker. The activated beads were then washed twice with a borate buffer. The activated beads were re-suspended in 200 microliters of borate buffer and mixed with 15 microliters of monoclonal antibody of C-reactive protein (CRP Mab 5811 from Biogenisis, Inc.) having a concentration of 6.4 milligrams per milliliter. The reaction was allowed to occur overnight on a shaker at room temperature. The resulting reaction mixture was then mixed with 100 microliters of ethanolamine for 15 minutes with a shaker. The supernatant solution was discarded and the beads were washed twice with a PBS buffer and stored at 4°C in a storage buffer that contained 0.1 molar PBS, 0.15 molar NaCl, 1% BSA, 5% glycerol and 0.1 % NaN₃ to result in conjugated beads of a 4 mg/ml concentration.

The fluorescent intensity was then measured at the detection/calibration line using a Fluorolog III Spectrofluoremeter (SPEX Industries, Inc., Edison, NJ) with a right angle mode. The results are shown below in Table 1, where Iₛ represents the signal intensity of the detection probes at the detection/calibration line and I_{c} represents the signal intensity of the calibration probes at the detection/calibration line.

**Table 1: Signal Intensity Results**

| Analyte (ng/ml) | I_{c} | Iₛ/I_{c} |
|---|---|---|
| 0 | 400 | 1258 |
| 33 | 426 | 1068 |
| 66 | 450 | 971 |
| 189 | 470 | 860 |
| 378 | 450 | 822 |
| 756 | 470 | 564 |

As evidenced from the results above, when no analyte was present in the sample, the detection probes moved pass the capture line and were captured on the detection/calibration line. When the analyte was present in the sample, the detection probes complexed with the analyte and were captured on the capture line, with any remaining detection probes being captured at the detection/calibration line. Therefore, the intensity of the detection probes (Iₛ) on the detection/catibration line was inversely proportional to the analyte concentration, while the intensity (I_{c}) of the calibration probes at the detection/calibration line remained relatively constant for different analyte concentrations, i.e., ~444 ± 27. Variations of I_{c} are believed to have been due to the varied environmental, assay conditions and instrumental instability. However, the variation is expected to have had a similar effect on Iₛ. Therefore Iₛ/I_{c} is believed to have provided a more accurate result in this instance.

### EXAMPLE 2

The ability of an internal detection/calibration zone of the present invention to calibrate a sandwich assay was demonstrated. Initially, HF 120 porous membrane samples were laminated onto corresponding supporting cards having a length of approximately 30 centimeters. Fluorescent polymers (calibration probes) were then formed as follows. Initially, 0.5 grams of polyacrylic acid (MW=450,000, D.P.= 6250) was dissolved into 20 milliliters of methanol. 3.8 milligrams of dicyclohexylcarbodiimide (DCC) in 1 milliliter of methanol was added to the solution to activate the polyacrylic acid polymer. The resulting solution was stirred for 30 minutes. A solution of fluorescent dye was made by dissolving 10 milligrams of 5-(and-6)-((N-(5-aminopentyl)amino)-carbonyl)tetramethylrhodamine (excitation/emission wavelengths of 544/590 nanometers, Molecular Probes, Inc.) into 1 milliliter of a methanol solution. The dye solution was added to the activated polyacrylic acid solution and stirred. The reaction was continued for 24 hours at room temperature with aluminum foil wrapped around the solution. After the reaction was stopped, diethylether was added to form a precipitate. The precipitate was centrifuged and soaked in ethanol to remove any unreacted fluorescent dye. After removing the ethanol solution, the residual red gel was dried in air.

The fluorescent polymers (calibration probes) described above were then dissolved in water and mixed with CelQuat® 100-H (a cellulosic polyelectrolytic derivative available from National Starch & Chemical, Inc.). This mixture was striped onto the membrane to form a detection/calibration line. Monoclonal antibody CRP Mab 5811 having a concentration of 6.4 milligrams per milliliter (obtained from Biogenesis, Inc.) was immobilized on the porous membrane samples to form a capture line. The membrane samples were then dried for 1 hour at a temperature of 37°C. A cellulosic fiber wicking pad (Millipore Co.) was attached to one end of the membrane and cut into 4-millimeter strips.

The half stick samples were put into various micro-wells in which mixtures of fluorescent beads (detection probes) and different concentrations of CRP antigen were contained. The amount of the fluorescent beads (detection probes) was the same for each mixture, i.e., 4 micrograms. The fluorescent beads (detection probes) were formed as follows. Initially, polystyrene beads having carboxyl functional groups (obtained from Molecular Probes, Inc.) were provided. The beads had a particle size of 0.2 micrometers and excitation/emission wavelengths of 505/515 nanometers. 100 microliters of the polystyrene beads (2% concentration) were washed twice with MES buffer and separated with a microcentrifuge. The pellitized beads were re-suspended in 200 microliters of MES buffer and mixed with 20 microliters of MES buffer containing 4.8 milligrams of carbodiimmide (from Polysciences, Inc.). The mixture was reacted at room temperature for 20 minutes on a shaker. The activated beads were then washed twice with a borate buffer. The activated beads were re-suspended in 200 microliters of borate buffer and mixed with 90 microliters of monoclonal antibody of C-reactive protein (CRP Mab 5804 from Biogenisis, Inc.) having a concentration of at 2.36 milligrams per milliliter. The reaction was allowed to occur overnight on a shaker at room temperature. The resulting reaction mixture was then mixed with 100 microliters of ethanolamine for 15 minutes with a shaker. The supernatant solution was discarded and the beads were washed twice with a PBS buffer and stored at 4°C in a storage buffer that contained 0.1 molar PBS, 0.15 molar NaCl, 1% BSA, 5% glycerol and 0.1 % NaN₃ to result in conjugated beads of a 4 mg/ml concentration.

The fluorescent intensity was then measured at the detection/calibration line using a Fluorolog III Spectrofluoremeter (SPEX Industries, Inc., Edison, NJ) with a right angle mode. The results are shown below in Table 2, where Iₛ represents the signal intensity of the detection probes at the detection/calibration line and I_{c} represents the signal intensity of the calibration probes at the detection/calibration line.

**Table 2: Signal Intensity Results**

| Analyte (ng/ml) | I_{c} | Iₛ/I_{c} |
|---|---|---|
| 0 | 37 | 16622 |
| 5 | 42 | 13714 |
| 25 | 36 | 15111 |
| 50 | 34 | 14971 |
| 250 | 34 | 9529 |
| 500 | 33 | 7152 |
| 2500 | 34 | 1765 |

As evidenced from the results above, when no analyte was present in the sample, the detection probes moved pass the capture line and were captured on the detection/calibration line. When the analyte was present in the sample, the detection probes complexed with the analyte and were captured on the capture line, with any remaining detection probes being captured at the detection/calibration line. Therefore, the intensity of the detection probes (Iₛ) on the detection/calibration line was inversely proportional to the analyte concentration, while the intensity (I_{c}) of the calibration probes at the detection/calibration line remained relatively constant for different analyte concentrations, i.e., ~36 ± 6. Variations of I_{c} are believed to have been due to the varied environmental, assay conditions and instrumental instability. However, the variation is expected to have had a similar effect on Iₛ. Therefore Iₛ/I_{c} is believed to have provided a more accurate result in this instance.

While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. A flow-through assay device capable of detecting the presence or quantity of an analyte residing in a test sample, said flow-through assay device comprising a porous membrane, said porous membrane being in communication with detection probes capable of generating a detection signal and calibration probes capable of generating a calibration signal, wherein the amount of the analyte is capable of determination from said detection signal as calibrated by said calibration signal, wherein said detection probes, said calibration probes, or combinations thereof, are conjugated with a specific binding member for the analyte, the flow-through assay device
**characterized by**
said porous membrane defining a detection/calibration zone within which a polyelectrolyte capture reagent is immobilized that is configured to directly or indirectly bind to the calibration probes, and wherein a second capture reagent is immobilized on said porous membrane within said detection/calibration zone, wherein said second capture reagent is configured to directly or indirectly bind to said detection probes, wherein said detection probes are capable of generating a detection signal and said calibration probes are capable of generating a calibration signal while within said detection/calibration zone.

2. A flow-through assay device as defined in claim 1, wherein the detection/calibration zone is a single detection/calibration zone.

3. A flow-through assay device as defined in claim 1 or 2, wherein the polyelectrolyte capture reagent is configured to directly or indirectly bind to the calibration probes through ionic interaction.

4. A flow-through assay device as defined in one of the preceding claims, wherein the second capture reagent is separately immobilized within the detection/calibration zone.

5. A flow-through assay device as defined in claim 1, wherein said detection probes and said calibration probes are selected from the group consisting of chromogens, catalysts, fluorescent compounds, chemiluminescent compounds, phosphorescent compounds, radioactive compounds, direct visual labels, liposomes, and combinations thereof.

6. A flow-through assay device as defined in claim 5, wherein said detection probes and said calibration probes are fluorescent compounds.

7. A flow-through assay device as defined in claim 1, wherein said detection probes, said calibration probes, or combinations thereof, contain microparticles.

8. A flow-through assay device as defined in claim 1, wherein the second capture reagent is a specific binding member for the analyte.

9. A flow-through assay device as defined in claim 1, wherein said polyelectrolyte has a net positive charge.

10. A flow-through assay device as defined in claim 9, wherein said polyelectrolyte is selected from the group consisting of polylysine, polyethylenimine, epichlorohydrin-functionalized polyamines or polyamidoamines, polydiallyldimethyl-ammonium chloride, cationic cellulose derivatives, and combinations thereof.

11. A flow-through assay device as defined in claim 1, wherein said polyelectrolyte has a net negative charge.

12. A flow-through assay device as defined in claim 1, wherein said polyelectrolyte is amphiphilic.

13. A flow-through assay device as defined in claim 1, wherein said detection probes do not have a charge that is opposite to the charge of the polyelectrolyte.

14. A flow-through assay device as defined in claim 1, wherein the device is a sandwich-type assay device.

15. A flow-through assay device as defined in claim 1, wherein the device is a competitive-type assay device.

16. A flow-through assay device as defined in claims 1 -15, wherein the polyelectrolyte capture reagent is non-diffusively immobilized.

17. A flow-through assay device as defined in claim 1, wherein the amount of the analyte within the test sample is proportional to the intensity of the detection signal divided by the intensity of the calibration signal.

18. A method for detecting the presence or quantity of an analyte residing in a test sample, said method comprising:
i) providing the flow-through assay device according to claim 1;
ii) contacting a test sample containing the analyte with said detection probes and said calibration probes;
iii) measuring the intensity of the detection signal and the intensity of the calibration signal generated within said detection/calibration zone; and
iv) calibrating the intensity of the detection signal with the calibration signal, wherein the amount of the analyte within the test sample is determined from said detection signal as calibrated by said calibration signal.

19. A method as defined in claim 18, wherein said detection probes and said calibration probes are selected from the group consisting of chromogens, catalysts, fluorescent compounds, chemiluminescent compounds, phosphorescent compounds, radioactive compounds, direct visual labels, liposomes, and combinations thereof.

20. A method as defined in claim 18, wherein said detection probes and said calibration probes are fluorescent compounds.

21. A method as defined in claim 20, further comprising exciting said detection probes and said calibration probes within said detection/calibration zone, wherein the excitation causes said detection probes to emit the detection signal and said calibration probes to emit the calibration signal.

22. A method as defined in claim 18, further comprising generating a calibration curve by plotting the intensity of the detection signal calibrated by the intensity of the calibration signal for a plurality of predetermined analyte concentrations.
